# EUROPEAN PATENT APPLICATION

(11) **EP 1 623 664 A1**
(43) Date of publication of application: **08.02.2006**
(21) Application number: 04730719.4
(22) Date of filing: 30.04.2004
(51) Int. Cl.: A61B 1/06

(54) **ENDOSCOPE SYSTEM**

(30) Priority: 14.05.2003 JP 2003136392
(71) Applicant: Olympus Corporation, Tokyo 151-0072 (JP)
(72) Inventor: KOITABASHI, Masanobu, Olympus Corporation, Tokyo 151-0072 (JP)
(74) Representative: von Hellfeld, Axel
(86) International application number: PCT/JP2004/006318
(87) International publication number: WO 2004/100777

(57) **Abstract**

An endoscope system 1 has a configuration including an endoscope main body 2, a light source 3 serving as an external apparatus having a light source lamp, and a suction device, a liquid forward-spraying device, and a liquid spraying tank 33 constituting a fluid apparatus. The endoscope system 1 has a configuration including a general connector 29 disposed in the endoscope main body 2, a light guide serving as a fluid path and a light transmission portion disposed in the endoscope main body 2, a light guide connector 52 serving as a light incident portion, a fluid connector 53 serving as a fluid path opening, a general connector receptacle portion 40 disposed in the light source 3, a condenser lens serving as a light guide portion, and tubes 31a to 33a of the suction device and the like and in-unit tubes in a channel unit constituting a fluid supply channel.

## Description

### Technical Field

The present invention relates to an endoscope system provided with an endoscope main body having a configuration capable of observing the inside of a subject.

### Background Art

In recent years, endoscope systems have been used widely. With respect to the endoscope system, a slender insertion portion is inserted into body cavities and, thereby, intracavital organs and the like are observed. If necessary, therapeutic instruments inserted into a therapeutic instrument insertion channel are used, so that various therapies and treatments can be conducted.

In general, the endoscope system is used by connecting an endoscope main body to external apparatuses, e.g., a light source and a control apparatus. In such a known endoscope system, the connection to the external apparatus is conducted, for example, via a connector disposed at an end portion of a universal cord or the like extended from a control section of the endoscope main body, as described in, for example, Japanese Unexamined Patent Application Publication No. 10-276963 and Japanese Unexamined Patent Application Publication No. 11-253393.

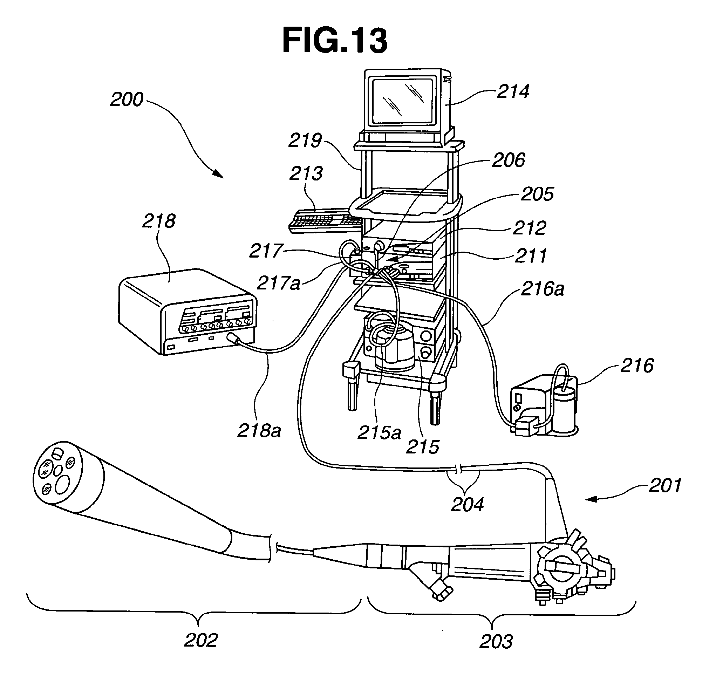

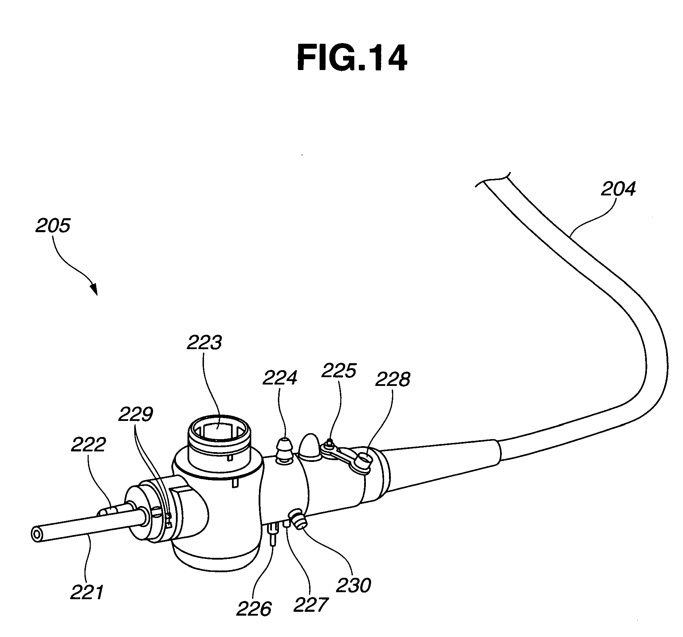

The known endoscope system has a configuration as shown in, for example, Fig. 13 and Fig. 14.

As shown in Fig. 13, the known endoscope system 200 has a configuration including an endoscope main body 201. The endoscope main body 201 comprises an insertion portion 202 and a control section 203. A universal cord 204 is extended from a side portion of the control section 203.

A connector 205 to be connected to a light source 211 is disposed at the end portion of the universal cord 204. An electrical cable 206 to be connected to a video processor 212 is extended from the side portion of the connector 205. A keyboard 213 to, for example, input patient information and a monitor 214 to display endoscope images are connected to the video processor 212.

Tubes, e.g., a discharge tube 215a of a suction device 215, a forward-spraying-liquid conducting tube 216a of a liquid forward-spraying device 216, and a tank tube 217a of a liquid spraying tank 217, are to be connected to this connector 205.

Furthermore, an earth cord 218a of a high-frequency cauterization apparatus 218 is to be connected to the connector 205. The light source 211, the video processor 212, the monitor 214, the suction device 215, and the like are placed on a rack 219 together with the keyboard 213.

As shown in Fig. 14, a light guide base 221 and an air blowing connector 222 are protruded from the connector 205. The light guide base 221 is to be connected to the light source 211. The air blowing connector 222 is to be connected to an air blowing pump, although not shown in the drawing, disposed in the light source 211.

An electrical connector 223 is disposed on the connector 205. The electrical connector 223 is to be connected to the video processor 212 via the electrical cable 206.

In addition, the connector 205 is provided with a suction connector 224, a liquid forward-spraying connector 225, a liquid spraying connector 226, and a pressurizing connector 227. The suction connector 224 is to be connected to the discharge tube 215a of the suction device 215. The liquid forward-spraying connector 225 is to be connected to the forward-spraying-liquid conducting tube 216a of the liquid forward-spraying device 216. The liquid spraying connector 226 and the pressurizing connector 227 are to be connected to the tank tube 217a of the liquid spraying tank 217.

Reference numeral 228 denotes a cap to block the liquid forward-spraying connector 225 when the connector is not used. Reference numeral 229 denotes an electrical contact to be connected to the light source 211 to transmit dimmer signals and the like to the light source.

Furthermore, The connector 205 is provided with an earth base 230 to which the earth cord 218a of the high-frequency cauterization apparatus 218 is to be connected.

The known endoscope system 200 including such an endoscope main body 201 must be cleaned and disinfected after each endoscopy. Consequently, the known endoscope system 200 exhibits a poor operation efficiency and is complicated since each of tubes and cords connected to the connector 205 of the endoscope main body 201 must be removed and attached every inspection.

On the other hand, in a known endoscope system, a video processor is connected to a light source by a connection cable, as described in, for example, Japanese Unexamined Patent Application Publication No. 2002-34912.

In the endoscope system described in the above-described publication, a connector of an endoscope main body is provided with an electrical contact portion. Consequently, in the endoscope system described in the above-described publication, the connector of the endoscope main body is connected to the light source by one action and, thereby, the endoscope main body can be connected to the video processor via this light source.

However, in the above-described publication, connections of an earth cord and tubes for, e.g., air/liquid supply and liquid forward-spraying, in the endoscope system are not referred to.

On the other hand, in the configuration of a known endoscope system, a light source and an air blowing pump are incorporated in a video processor and are integrated, as described in, for example, Japanese Unexamined Patent Application Publication No. 62-34525.

In the endoscope system described in the above-described publication, the connector of the endoscope main body is connected to the video processor by one action and, thereby, an electrical connector, a light guide connector, and an air blowing connector are connected at a time.

In the endoscope system described in the above-described Japanese Unexamined Patent Application Publication No. 62-34525, the air blowing connector to be connected to an air blowing pump is disposed, but connections of other tubes, e.g., a liquid spraying tube and a spraying-liquid conducting tube, and an earth cord, are not referred to.

Furthermore, in a known endoscope system, the connection can be conducted in either case where a video processor and a light source are integrated or separated, as described in, for example, US Patent No. 5,239,983.

The present invention was made in consideration of the above-described problems. Accordingly, it is an object of the present invention to provide an endoscope system in which tubes and cords can be connected to an endoscope main body by one action, and excellent operability is exhibited.

### Disclosure of Invention

An endoscope system of the present invention includes:
an endoscope main body having a configuration capable of observing the inside of a subject;
an external apparatus including a light source to emit illumination light for illuminating an inspection area of the subject;
a fluid apparatus to supply a fluid to be ejected from the endoscope main body or to suction a fluid in the endoscope main body, the fluid apparatus disposed separately from the external apparatus;
a connector to connect the endoscope main body to the external apparatus, the connector disposed on one end portion side of the endoscope main body;
a light transmission portion to transmit the light incident on the endoscope main body via the connector to the other end portion side of the endoscope main body, the light transmission portion disposed in the endoscope main body;
a fluid path to allow the fluid to pass through the endoscope main body via the connector, the fluid path disposed in the endoscope main body;
a connector receptacle to connect the endoscope main body to the external apparatus by the connector being inserted into the connector receptacle disposed in the external apparatus;
a light guide portion to guide the illumination light from the light source to the connector receptacle;
a light incident portion which is optically connected to the light guide portion in accordance with an action to insert the connector into the connector receptacle and which is disposed on one end portion side of the light transmission portion;
a fluid path opening disposed to allow the fluid supplied from the fluid apparatus or the fluid suctioned by the fluid apparatus to flow into the fluid path in accordance with an action to insert the connector into the connector receptacle, the fluid path opening disposed on one end portion side of the fluid path; and
a fluid supply channel which constitutes a flow channel to allow the fluid supplied from the fluid apparatus or the fluid suctioned by the fluid apparatus to flow into the fluid path opening via the external apparatus by being connected to the fluid path opening in accordance with an action to insert the connector into the connector receptacle.

### Brief Description of the Drawings

Fig. 1 is an entire configuration diagram showing an endoscope system according to an embodiment of the present invention.
Fig. 2 is an explanatory diagram showing a connection relationship between a light source and a video processor shown in Fig. 1.
Fig. 3 is an explanatory diagram showing a flow path in an endoscope main body shown in Fig. 1.
Fig. 4 is a circuit block diagram showing an internal configuration of the light source and the video processor shown in Fig. 2.
Fig. 5 is a magnified perspective view showing a general connector receptacle portion and its periphery in the light source shown in Fig. 2.
Fig. 6 is a perspective view showing the back side of a channel unit shown in Fig. 5.
Fig. 7 is a front view showing the general connector receptacle portion and its periphery in the light source shown in Fig. 5.
Fig. 8 is an explanatory diagram showing a channel unit mounting portion and its periphery in the inside of the light source shown in Fig. 5 and an internal configuration of the channel unit.
Fig. 9 is a sectional view of a key portion showing a removable attachment mechanism and its periphery in the light source.
Fig. 10 is a vertical sectional view of the removable attachment mechanism and its periphery shown in Fig. 9.
Fig. 11 is an explanatory diagram showing the operation of the present embodiment.
Fig. 12 is an explanatory diagram showing an air/liquid supply control valve and its periphery.
Fig. 13 is an entire configuration diagram showing a known endoscope system.
Fig. 14 is an external perspective view showing connectors of an endoscope main body shown in Fig. 13.

### Best Mode for Carrying Out the Invention

An embodiment of the present invention will be described below with reference to drawings.

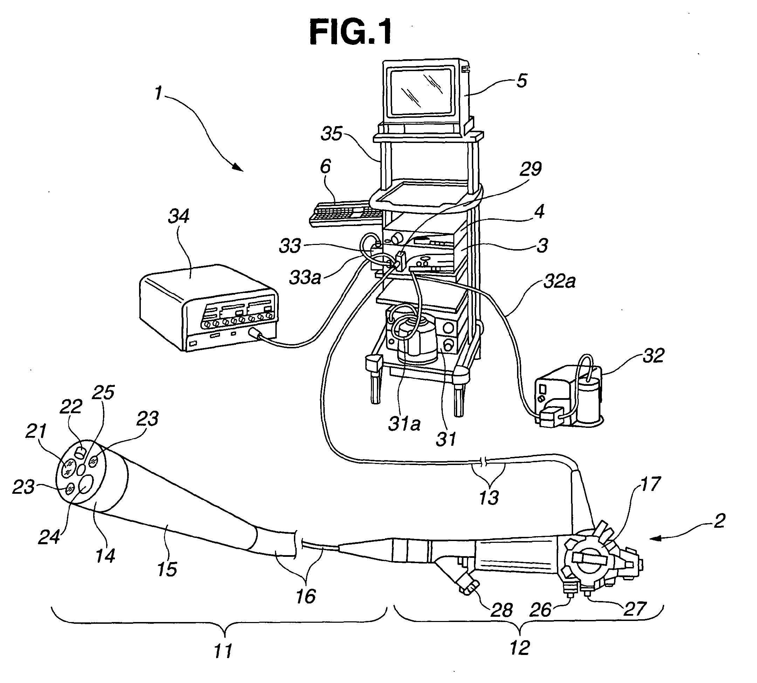

Fig. 1 to Fig. 12 show one embodiment of an endoscope system according to the present invention.

As shown in Fig. 1, the endoscope system 1 of the embodiment of the present invention has a configuration including an endoscope main body 2 having a configuration capable of observing the inside of a subject, a light source 3 serving as an external apparatus to supply illumination light, and a video processor 4 to conduct signal processing on output signals output from an image pickup portion of the endoscope main body 2 and to output video signals.

The video processor 4 is connected to a monitor 5. The video processor 4 outputs video signals to the monitor 5 so as to display endoscope images. A keyboard 6 to input patient information and the like is connected to the video processor 4.

The endoscope main body 2 includes a slender insertion portion 11 to be inserted into body cavities and the like and a control section 12 disposed continuously on the proximal end side of this insertion portion 11, also serving as a grip section.

The endoscope main body 2 is provided with a soft universal cord 13 extended from a side portion of the control section 12. The universal cord 13 includes a light guide serving as a light transmission portion, signal cables, and tubes and cables constituting a fluid path, as described below, in the inside.

The insertion portion 11 is configured to have a hard distal end portion 14 disposed at the distal end of the insertion portion 11, a freely bendable bending section 15 disposed at a rear end of the distal end portion 14, and long flexible tube section 16 having flexibility disposed at a rear end of this bending section 15.

The bending section 15 can be bended by the operation of the bend control lever 17 disposed in the control section 12.

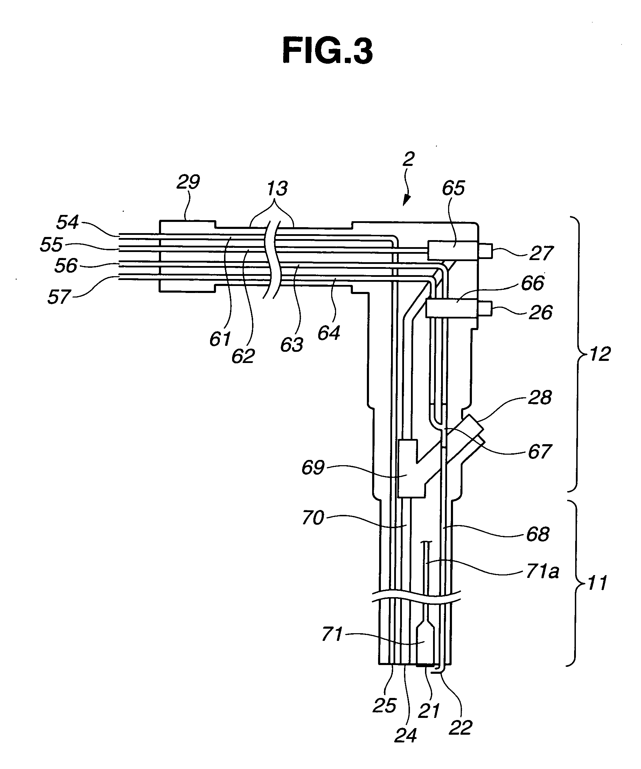

The distal end portion 14 of the insertion portion 11 is provided with an observation window 21, an air/liquid supply nozzle 22 to eject a fluid, e.g., liquid or air, toward the surface of this observation window 21 so as to conduct cleaning, an illumination window 23, a distal end opening 24 of a therapeutic instrument insertion channel (refer to Fig. 3), and a liquid forward-spraying opening 25 to clean an inspection area of a subject.

An objective optical system, although not shown in the drawing, is disposed in a rear direction of the observation window 21. Although not shown in the drawing, an image pickup surface (in the case of an optical endoscope, an image entry surface of an image transmission optical system, e.g., an image guide and a relay lens) of an image pickup unit is disposed at the position of image formation of this objective optical system. An exit surface of the light guide is disposed in a rear direction of the illumination window 23.

The control section 12 is provided with an air/liquid supply operation button 26 and a suction operation button 27. The air/liquid supply operation button 26 is an operation button to eject a fluid, e.g., a gas and a liquid, from the air/liquid supply nozzle 22. The suction button 27 is an operation button to selectively recover intracavital mucus and the like through the distal end opening 24 of the therapeutic instrument insertion channel.

In the therapeutic instrument insertion channel, a fluid path is formed by tubes and the like, as described below, disposed in the insertion portion 11. This therapeutic instrument insertion channel is communicated with a therapeutic instrument insertion hole 28 disposed in the vicinity of the front end of the control section 12. A forceps valve, although not shown in the drawing, is removably attached on an end edge portion of the therapeutic instrument insertion hole 28.

A general connector 29 to be connected to the light source 3 is disposed at the end portion of the universal cord 13.

On the other hand, the general connector 29 is connected to the light source 3. Furthermore, tubes, e.g., a discharge tube 31a of a suction device 31, a forward-spraying-liquid conducting tube 32a of a liquid forward-spraying device 32, and a tank tube 33a of a liquid spraying tank 33, are to be connected to the light source 3. The suction device 31, the liquid forward-spraying device 32, and the liquid spraying tank 33 constitute the fluid apparatus.

The light source 3, the video processor 4, the monitor 5, the suction device 31, and the like are placed on a rack 35 together with the keyboard 6.

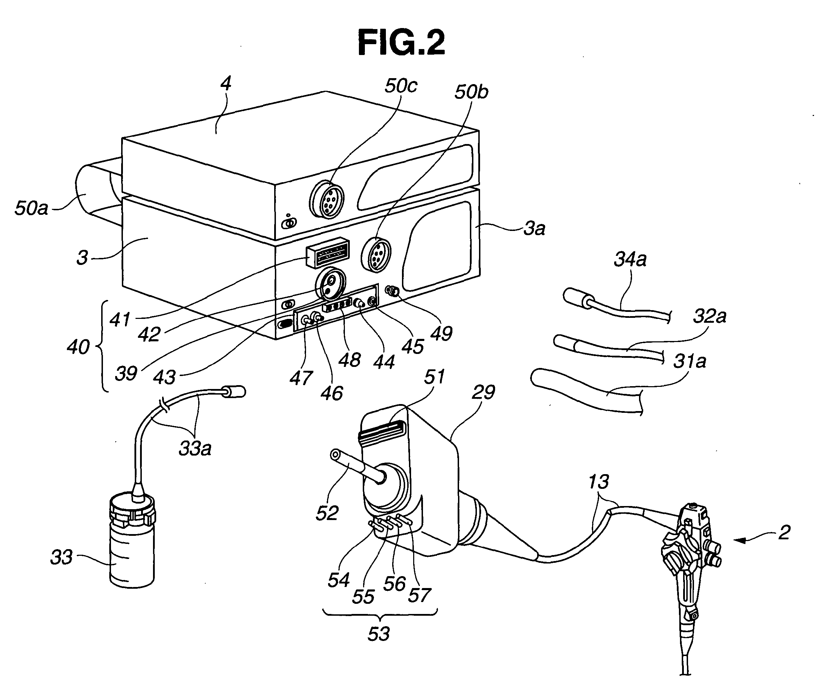

As shown in Fig. 2, tubes 31a to 33a of the suction device 31 and the like are removably connected to the front panel 3a of the light source 3. In addition, a general connector receptacle portion 40, to which the general connector 29 of the endoscope main body 2 is removably connected, is disposed on the front panel 3a of the light source 3.

The general connector receptacle portion 40 has a configuration including an electrical connector receptacle portion 41, a light guide receptacle portion 42, and a channel unit portion 43.

An air blowing connector receptacle portion 39 is disposed under the light guide receptacle portion 42. In this manner, the light source 3 can be used while the air blowing connector 222 (refer to Fig. 14) disposed on the connector of the known endoscope main body is connected thereto.

The channel unit portion 43 is provided with a suction connector 44, a liquid forward-spraying connector 45, a liquid spraying connector 46, and a pressurizing connector 47.

The suction connector 44 is to be connected to the discharge tube 31a of the suction device 31. The liquid forward-spraying connector 45 is to be connected to the forward-spraying-liquid conducting tube 32a of the liquid forward-spraying device 32. The liquid spraying connector 46 and the pressurizing connector 47 are to be connected to the tank tube 33a of the liquid spraying tank 33. The electrical connector receptacle portion 41 constitutes a second electrical connection portion.

The channel unit portion 43 is provided with a fluid connector receptacle portion 48. The fluid connector receptacle portion 48 is provided with a liquid forward-spraying connector receptacle portion, a suction connector receptacle portion, a pressurizing connector receptacle portion, and a liquid spraying connector receptacle portion, as described below. The channel unit portion 43 has a configuration in which the channel unit, as described below, can be removably attached to the channel unit mounting portion.

The front panel 3a of the light source 3 is provided with an earth base 49 to which an earth cord 34a of a high-frequency cauterization apparatus 34 is to be connected. An electrocautery or a high-frequency probe, although not shown in the drawing, is connected to the high-frequency cauterization apparatus 34 and, thereby, an affected area of a patient can be subjected to a high-frequency treatment.

The light source 3 is connected to the video processor 4 by an electrical cable 50a extended from the back. These light source 3 and the video processor 4 are provided with electrical connectors 50b and 50c. In this manner, these light source 3 and the video processor 4 can be used while the electrical connector 223 disposed on the connector 205 of the known endoscope main body 201 illustrated in Fig. 13 and Fig. 14 is connected thereto via the electrical cable 206.

On the other hand, the general connector 29 is provided with an electrical connector 51, a light guide connector 52, and a fluid connector 53.

The electrical connector 51 is to be connected to the electrical connector receptacle portion 41 of the general connector receptacle portion 40. The light guide connector 52 is to be connected to the light guide receptacle portion 42 of the general connector receptacle portion 40. The fluid connector 53 is to be connected to the fluid connector receptacle portion 48 of the general connector receptacle portion 40.

The electrical connector 51 constitutes a first electrical connection portion, the light guide connector 52 constitutes a light incident portion, and the fluid connector 53 constitutes a fluid path opening.

The fluid connector 53 is provided with a liquid forward-spraying connector 54, a suction connector 55, a pressurizing connector 56, and a liquid spraying connector 57.

In the endoscope main body 2, tubes communicated with the general connector receptacle portion 40 constitute a fluid path, as shown in Fig. 3.

A liquid forward-spraying tube 61 is connected to the liquid forward-spraying connector 54. A suction tube 62 is connected to the suction connector 55. An air blowing tube 63 is connected to the pressurizing connector 56. A liquid spraying tube 64 is connected to the liquid spraying connector 57. All these liquid forward-spraying tube 61, suction tube 62, air blowing tube 63, and liquid spraying tube 64 are led into the control section 12 through the inside of the universal cord 13.

In the inside of the control section 12, a suction control valve 65 is interposed at the midpoint of the suction tube 62, and an air/liquid supply control valve 66 is interposed at the midpoints of the air blowing tube 63 and the liquid spraying tube 64.

The distal end of the air blowing tube 63 and the distal end of the liquid spraying tube 64 are joined into an air/liquid supply tube 68 constituting one air/liquid supply channel via an air/liquid supply channel branch portion 67 in the inside of the control section 12. The air/liquid supply channel branch portion 67 is formed into the shape of a letter Y.

This air/liquid supply tube 68 is led to the distal end portion 14 through the inside of the insertion portion 11, and is connected to an air/liquid supply nozzle 22.

In the endoscope main body 2, switching of the air blowing channel and the liquid spraying channel and opening and closing thereof are conducted by an pressing operation of an air/liquid supply operation button 26. In this manner, in the endoscope main body 2, air blowing or liquid spraying to one air/liquid supply tube 68 can be conducted selectively via the air/liquid supply channel branch portion 67.

Therefore, the endoscope main body 2 can conduct cleaning by ejecting a fluid, e.g., liquid or air, from the air/liquid supply nozzle 22 toward the observation window 21.

On the other hand, the distal end of the suction tube 62 is connected to the midpoint of a therapeutic instrument insertion channel 70 via a suction channel branch portion 69 substantially in the shape of a letter Y. The suction tube 62 and the distal end side of the therapeutic instrument insertion channel 70 constitute a suction channel.

The proximal-side portion, which does not constitute the suction tube, of the therapeutic instrument insertion channel 70 constitutes a therapeutic instrument insertion hole 28.

In the control section 12, the suction control valve 65 having a suction operation button 27 is interposed at the midpoint of the suction tube 62. In general, this suction control valve 65 interrupts the suctioning action by closing the suction tube 62. When the suction operation button is pressed down, the suction tube 62 is opened by the action of the suction control valve 65 and the suctioning action through the suction tube 62 exerts an effect on the therapeutic instrument insertion channel 70 in the endoscope main body 2. In this manner, the endoscope main body 2 can suction and recover intracavital mucus and the like from the distal end opening 24 of the suction tube.

The suction control valve 65 has a configuration capable of suctioning the outside air to reduce a load on the suction pump when the suction tube 62 is closed and the effect of the suctioning action on the therapeutic instrument insertion channel 70 is interrupted.

In the inside of the endoscope main body 2, the liquid forward-spraying tube 61 is composed of one tube up to a liquid forward-spraying opening 25. The liquid forward-spraying tube 61 sprays forward sterilized water supplied by the operation of the liquid forward-spraying device 32 toward the inspection area from the liquid forward-spraying opening 25.

In Fig. 3, reference numeral 71 denotes an image pickup unit. A signal cable 71a extended from this image pickup unit 71 is connected to an electrical connector 51 (refer to Fig. 2) of the general connector 29.

The general connector 29 of the endoscope main body 2 is connected to the general connector receptacle portion 40 of the light source 3 and, thereby, the endoscope main body 2 is connected to the video processor 4, the suction device 31, the liquid forward-spraying device 32, and the earth of the high-frequency cauterization apparatus 34 via the light source 3.

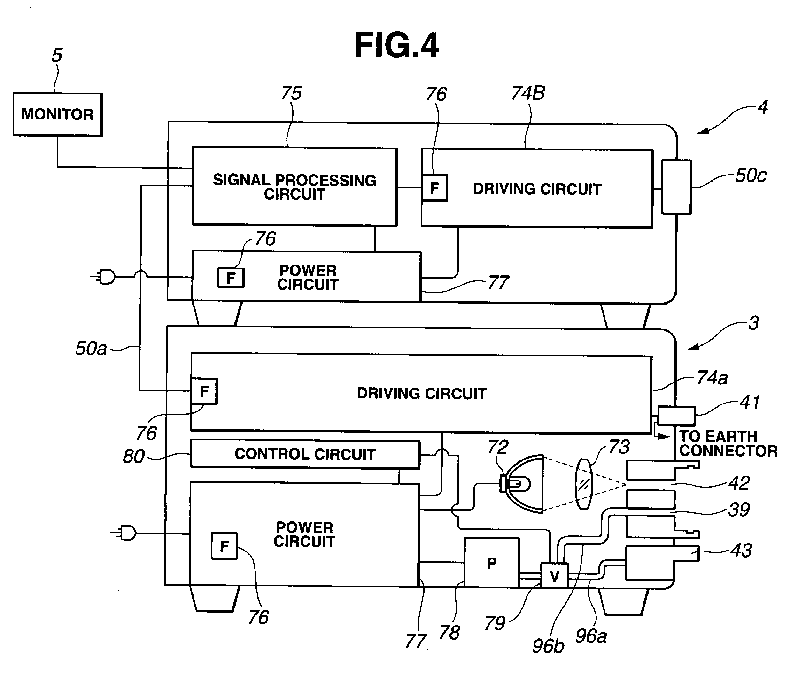

As shown in Fig. 4, the light source 3 is provided with a light source lamp 72 to emit illumination light for illuminating the inspection area of the subject.

When the light guide connector 52 of the endoscope main body 2 is connected to the light guide receptacle portion 42 of the light source 3, the illumination light emitted from the light source lamp 72 is concentrated and incident on a light entry surface of the light guide, although not shown in the drawing, in the light guide connector 52 by a condenser lens 73 serving as a light guide portion and the light is led to a light exit terminal.

The illumination light transmitted from the light guide illuminates the inspection area, e.g., an affected area, through the illumination window 23 of the insertion portion distal end portion 14 via an illuminational optical system, although not shown in the drawing.

The reflected light from the inspection area illuminated by this illumination light is taken into the endoscope main body 2 as a subject image through the observation window 21 of the insertion portion distal end portion 14 via an objective optical system.

The light source 3 has a configuration including a driving circuit 74A. The driving circuit 74A drives the image pickup unit 71 of the endoscope main body 2. The image pickup unit 71 is controlled and driven by the driving circuit 74A of the light source 3.

The video processor 4 has a configuration including a driving circuit 74B similar to that of the light source 3 in order that a known endoscope main body can be connected and used.

The driving signals output from the driving circuit 74A of the light source 3 via the electrical connector receptacle portion 41 are transmitted to the signal cable 71a via the electrical connector 51 of the general connector 29, and drive the image pickup unit 71.

The subject image taken into the endoscope main body 2 is picked up and subjected to photoelectrical conversion by the image pickup unit 71, so as to be converted into an image pickup signal. This image pickup signal is transmitted from the image pickup unit 71 through the signal cable 71a.

The image pickup signal transmitted through the signal cable 71a is output from the electrical connector 51 of the universal cord 13 to the driving circuit 74A of the light source 3 via the electrical connector receptacle portion 41. The image pickup signal is output from the driving circuit 74A of the light source 3 to a signal processing circuit 75 of the video processor 4. The signal processing circuit 75 subjects the image pickup signal to signal processing, and generates a standard video signal. The signal processing circuit 75 outputs the generated video signal to the monitor 5, and this monitor 5 is allowed to display an endoscope image.

The light source 3 and the video processor 4 are provided with isolation circuits 76 (indicated by "F" in the drawing) in the driving circuits 74A and 74B, respectively. These isolation circuits 76 are composed of isolation elements, e.g., a photocoupler or a capacitor, a transformer, or the like, although not shown in the drawing.

Here, the driving circuits 74A and 74B are patient circuits connected to the image pickup unit 71 in the endoscope main body 2. These driving circuits 74A and 74B are configured to be isolated by the isolation circuits 76 so that electrical signals can be transmitted while electrical insulation from the signal processing circuit 75 and other circuits in the apparatus is maintained with predetermined withstand voltage and leakage current.

The light source 3 and the video processor 4 have configurations including respective power circuits 77 to be connected to supply mains. In this power circuits 77 as well, isolation circuits 76 are disposed as in the driving circuits 74A and 74B. In this manner, the power circuits 77 have configurations capable of transmitting the power to each part while electrical insulation from the supply mains is maintained with predetermined withstand voltage and leakage current.

The light source 3 has a configuration including an air blowing pump 78, an air blowing valve 79 disposed downstream from this air blowing pump 78, and a control circuit 80 to control the switching (open and close) of this air blowing valve 79. The blowing gas from the air blowing pump 78 is supplied to the air blowing connector receptacle portion 39 or the channel unit portion 43 by being switched with the air blowing valve 79.

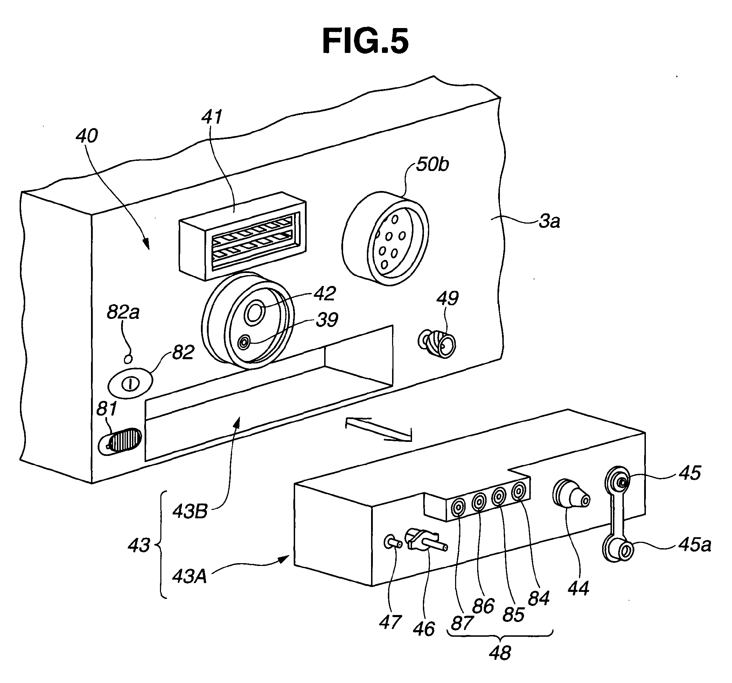

The detailed configuration of the general connector receptacle portion 40 of the light source 3 will be described below with reference to Fig. 5 to Fig. 8.

As shown in Fig. 5, a channel unit locking release knob 81 is disposed on a front panel 3a in the general connector receptacle portion 40 of the light source 3.

The channel unit portion 43 has a configuration in which the channel unit 43A is removably attached to the channel unit mounting portion 43B by operating the channel unit locking release knob 81.

Reference numeral 82 denotes a power switch of the light source 3. Reference numeral 82a denotes a power operating status indicating portion formed from an LED (Light Emitting Diode) or the like.

The power operating status indicating portion 82a lights up to make a notification to the operator when the power switch 82 is on and the light source 3 is in operation.

As described above, the channel unit 43A is provided with the suction connector 44, the liquid forward-spraying connector 45, the liquid spraying connector 46, and the pressurizing connector 47, as well as the fluid connector receptacle portion 48. A cap 45a is attached to the liquid forward-spraying connector 45 in order to block the connector when it is not used.

The fluid connector receptacle portion 48 is to be connected to the fluid connector 53 of the general connector 29. The fluid connector receptacle portion 48 is provided with the liquid forward-spraying connector receptacle portion 84, the suction connector receptacle portion 85, the pressurizing connector receptacle portion 86, and the liquid spraying connector receptacle portion 87.

The liquid forward-spraying connector receptacle portion 84 is to be connected to the liquid forward-spraying connector 54 of the fluid connector 53. The suction connector receptacle portion 85 is to be connected to the suction connector 55 of the fluid connector 53. The pressurizing connector receptacle portion 86 is to be connected to the pressurizing connector 56 of the fluid connector 53. The liquid spraying connector receptacle portion 87 is to be connected to the liquid spraying connector 57 of the fluid connector 53.

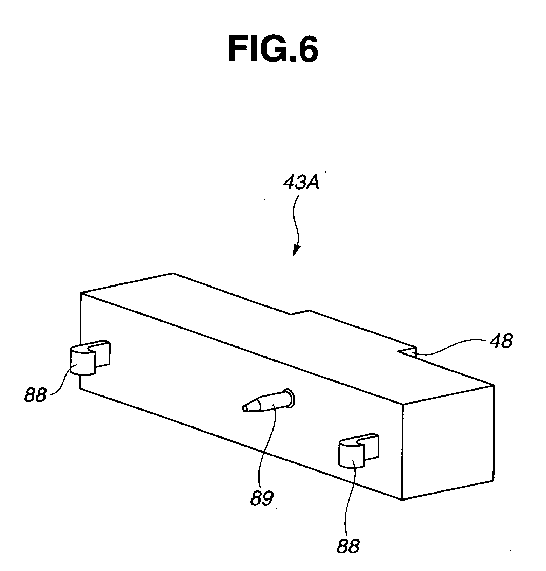

As shown in Fig. 6, locking claws 88 are disposed on the back side of the channel unit 43A. The channel unit 43A is removably attached to the channel unit mounting portion 43B by being locked with the locking claws 88.

In addition, an air blowing input connector 89 is disposed on the back side of the channel unit 43A. This air blowing input connector 89 inputs a blowing gas supplied from the air blowing pump 78 via the air blowing valve 79.

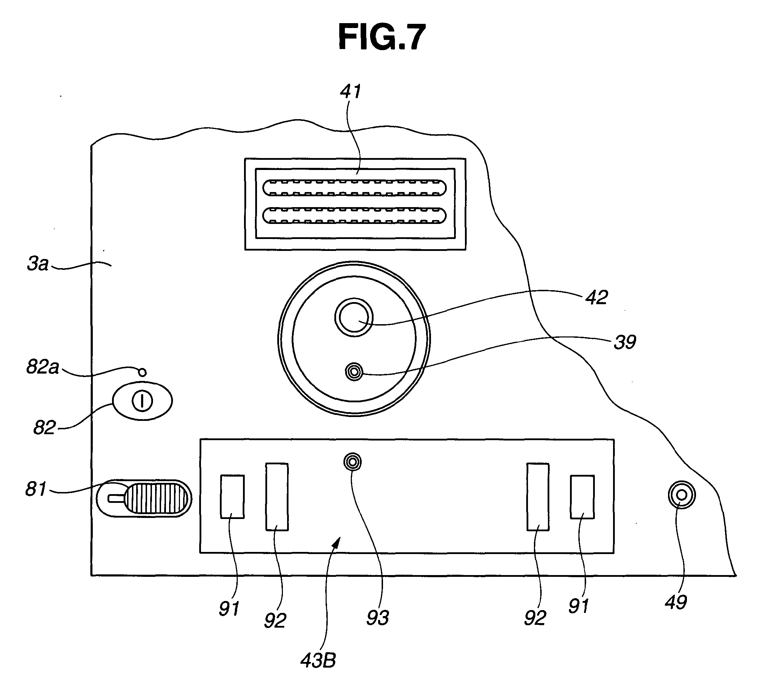

On the other hand, as shown in Fig. 7, the channel unit mounting portion 43B is provided with locking claw insertion holes 91. In addition, the channel unit mounting portion 43B is provided with a removable attachment mechanism, as described below, in the inside.

The channel unit mounting portion 43B releasably locks the channel unit 43A with the removable attachment mechanism by the locking claws 88 being inserted into the locking claw insertion holes 91.

The channel unit mounting portion 43B is provided with protruding portions 92 to facilitate positioning in attachment of the channel unit 43A. Furthermore, the channel unit mounting portion 43B is provided with an air blowing input connector receptacle portion 93 to be connected to the air blowing input connector 89 of the channel unit 43A.

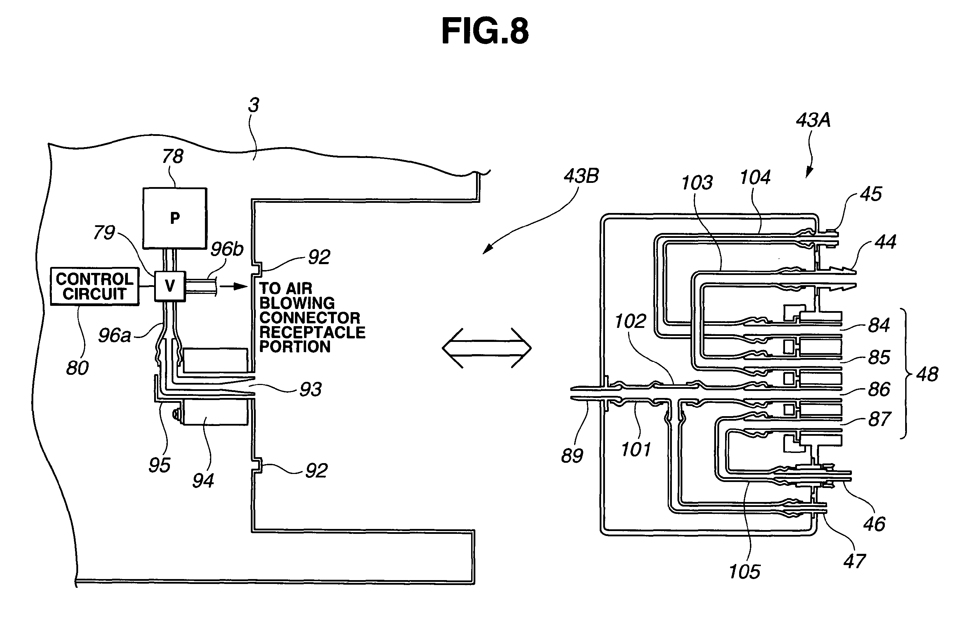

As shown in Fig. 8, the distal end side of the air blowing input connector receptacle portion 93 is held by an air blowing input connector receptacle holding portion 94 in the inside of the apparatus. The rear end side of the air blowing input connector receptacle portion 93 is held by an air blowing input connector holding plate 95. The air blowing input connector holding plate 95 is fixed by screws to the air blowing input connector receptacle holding portion 94.

The rear end side of the air blowing input connector receptacle portion 93 is connected to an apparatus-side first air blowing tube 96a extended from the air blowing valve 79. The blowing gas from the air blowing pump 78 is supplied to the air blowing input connector receptacle portion 93.

An apparatus-side second air blowing tube 96b extended from the air blowing valve 79 is connected to the air blowing connector receptacle portion 39 (refer to Fig. 7). The blowing gas from the air blowing pump 78 is supplied to the apparatus-side second air blowing tube 96b.

On the other hand, in the inside of the channel unit 43A, an in-unit air blowing tube 101 is connected to the distal end side of the air blowing input connector 89. The in-unit air blowing tube 101 is branched into the pressurizing connector 47 and a pressurizing connector receptacle portion 86 of the fluid connector receptacle portion 48 via an air-blowing and pressurizing branch portion 102. The air-blowing and pressurizing branch portion 102 is formed into the shape of a letter T.

In the inside of the channel unit 43A, the suction connector 44 and the suction connector receptacle portion 85 of the fluid connector receptacle portion 48 are joined by an in-unit suction tube 103.

As described above, the suction connector 44 is to be connected to the discharge tube 31a of the suction device 31. As described above, the suction connector receptacle portion 85 is to be connected to the suction connector 55 of the general connector 29.

Likewise, in the inside of the channel unit 43A, the liquid forward-spraying connector 45 and the liquid forward-spraying connector receptacle portion 84 of the fluid connector receptacle portion 48 are joined by an in-unit liquid forward-spraying tube 104.

As described above, the liquid forward-spraying connector 45 is to be connected to the forward-spraying-liquid conducting tube 32a of the liquid forward-spraying device 32. As described above, the liquid forward-spraying connector receptacle portion 84 is to be connected to the liquid forward-spraying connector 54 of the general connector 29.

Likewise, in the inside of the channel unit 43A, the liquid spraying connector 46 and the liquid spraying connector receptacle portion 87 are joined by an in-unit liquid spraying tube 105.

As described above, the liquid spraying connector 46 is to be connected to the tank tube 33a of the liquid spraying tank 33. As described above, the liquid spraying connector receptacle portion 87 is to be connected to the liquid spraying connector 57 of the general connector 29.

In this manner, the channel unit 43A can connect the endoscope main body 2 to various apparatuses, e.g., the suction device 31, via the in-unit tubes 103 to 105, since joining is conducted only in the inside of the unit without passing the liquids as fluids through the inside of the light source.

That is, the tubes 31a to 33a of the suction device 31 and the like and the in-unit tubes 103 to 105 in the channel unit 43A constitute a fluid supply channel.

The in-unit tubes 103 to 105 in the channel unit 43A constitute a first connection channel. The tubes 31a to 33a of the suction device 31 and the like constitute a second connection channel. The fluid connector receptacle portion 48 of the channel unit portion 43 constitutes a first opening. The liquid forward-spraying connector 45, the liquid spraying connector 46, and the pressurizing connector 47 constitute a second opening.

Therefore, the channel unit mounting portion 43B and the channel unit 43A require only the connection of the air blowing input connector 89. That is, the channel unit mounting portion 43B and the channel unit 43A require only the connection of the gas system among the fluids.

Consequently, the cleaning effectiveness and sterilization/disinfection effectiveness of the liquid channels can be improved since the channel unit 43A is taken off from the channel unit mounting portion 43B by using the removable attachment mechanism.

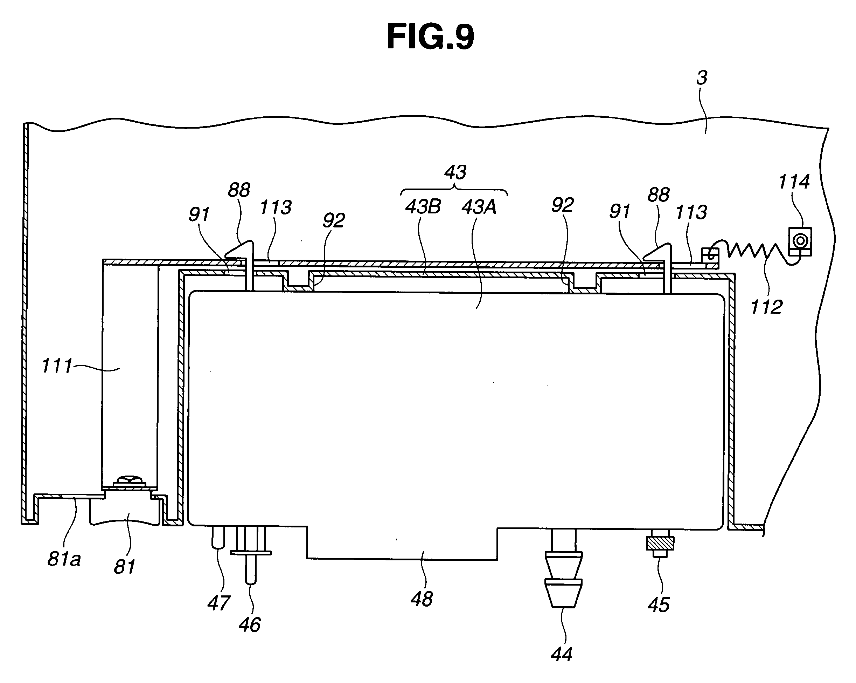

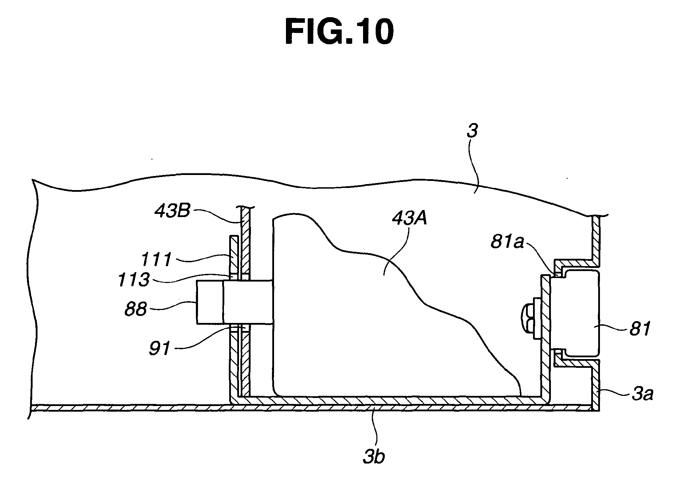

The removable attachment mechanism of the channel unit portion 43 will be described below with reference to Fig. 9 and Fig. 10.

As shown in Fig. 9 and Fig. 10, the removable attachment mechanism has a configuration including a channel unit locking piece 111 movable in a horizontal direction on the back side of the channel unit mounting portion 43B. In Fig. 10, reference numeral 3b denotes an outer covering member of the light source 3.

One end of the channel unit locking piece 111 is fixed by a screw to the channel unit locking release knob 81, and the other end is joined to a helical extension spring 112. This channel unit locking piece 111 is provided with locking claw holding holes 113. The locking claws 88 of the channel unit 43A, inserted through the locking claw insertion holes 91 of the channel unit mounting portion 43B, are hooked on the locking claw holding holes 113.

The channel unit locking release knob 81 is inserted through a knob hole 81a and exposed at the front panel 3a while being protruded and allowed to move in a horizontal direction. The channel unit locking piece 111 is fixed by a screw to the proximal end side of the channel unit locking release knob 81.

One end of the helical extension spring 112 is joined with the channel unit locking piece 111 and the other end is connected and fixed to a spring fixing member 114. Consequently, in general, the helical extension spring 112 is energized in a direction in which the positions of the locking claw insertion holes 91 of the channel unit mounting portion 43B and the locking claw holding holes 113 of the channel unit locking piece 111 become in a staggered configuration and the locking claws 88 of the channel unit 43A are held (locked), that is, in a right direction in Fig. 9, so as to lock the channel unit locking release knob 81.

According to the operation of the removable attachment mechanism, when the channel unit locking release knob 81 is moved in a left direction, in Fig. 9, against the energization force of the helical extension spring 112, the positions of the locking claw insertion holes 91 of the channel unit mounting portion 43B and the locking claw holding holes 113 of the channel unit locking piece 111 become in agreement, and therefore, the locking claws 88 of the channel unit 43A can be removably attached. In this manner, the channel unit 43A can be removed from and attached to the channel unit mounting portion 43B at will by the removable attachment mechanism through the operation of the channel unit locking release knob 81.

In the thus configured endoscope system 1, as described with reference to Fig. 1, the endoscope main body 2 is connected to the light source 3. And, the video processor 4, the suction device 31, the liquid forward-spraying device 32, the liquid spraying tank 33, and the high-frequency cauterization apparatus 34 are connected to this light source 3, and the endoscope system 1 is used for endoscopy and the like.

The operator connects the tubes and cords of suction device 31 and the like to the front panel 3a of the light source 3. First, the operator connects the tubes 31a to 33a of the suction device 31 and the like to the channel unit portion 43 of the general connector receptacle portion 40, as described with reference to Fig. 2.

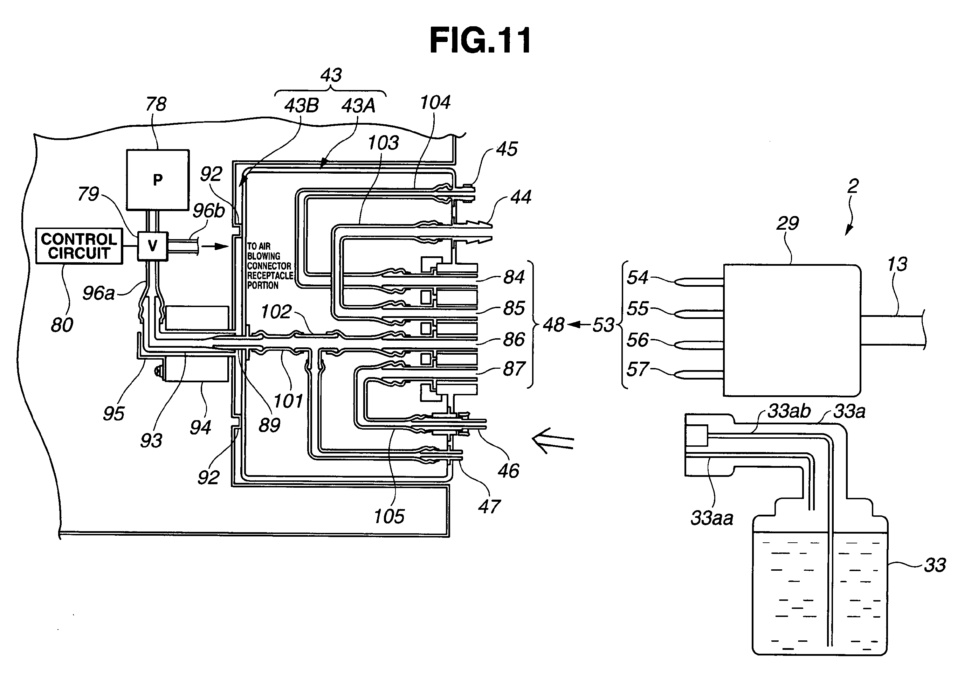

The operator connects the discharge tube 31a of the suction device 31 to the suction connector 44, connects the forward-spraying-liquid conducting tube 32a of the liquid forward-spraying device 32 to the liquid forward-spraying connector 45, and connects the tank tube 33a of the liquid spraying tank 33 to the liquid spraying connector 46 and the pressurizing connector 47. The pressurizing connector 47 is connected to a pressurizing tube 33aa of the tank tube 33a and, in addition, the liquid spraying connector 46 is connected to a liquid guide tube 33ab of the tank tube 33a (refer to Fig. 11). Furthermore, in the case where an affected area is subjected to a high-frequency treatment by using an electrocautery or the like, the operator connects the earth cord 34a of the high-frequency cauterization apparatus 34 to the earth base 49.

Subsequently, the operator connects the endoscope main body 2 to the light source 3. Here, the operator connects the general connector 29 of the endoscope main body 2 to the general connector receptacle portion 43 of the light source 3 by one action. Since the general connector 29 of the endoscope main body 2 is in correspondence with the general connector receptacle portion 43 of the light source 3, the electrical connector 51 is connected to the electrical connector receptacle portion 41, the light guide connector 52 is connected to the light guide receptacle portion 42, and the fluid connector 53 is connected to the fluid connector receptacle portion 48.

Here, in the connection between the fluid connector 53 and the fluid connector receptacle portion 48, as shown in Fig. 11, the liquid forward-spraying connector 54 is connected to the liquid forward-spraying connector receptacle portion 84, the suction connector 55 is connected to the suction connector receptacle portion 85, the pressurizing connector 56 is connected to the pressurizing connector receptacle portion 86, and the liquid spraying connector 57 is connected to the liquid spraying connector receptacle portion 87.

The operator inserts the insertion portion 11 of the endoscope main body 2 into body cavities and the like of the subject, and observes the inspection area.

Here, for example, in some cases, endoscopy with the endoscope main body 2 becomes difficult since the observation window 21 of the insertion portion distal end portion 14 gets soiled due to adhesion of body fluids.

At this time, the operator presses down the air/liquid supply operation button 26 of the endoscope control section 12 and, thereby, conducts cleaning by ejecting the fluid, e.g., a gas or a liquid, from the air/liquid supply nozzle 22 toward the surface of the observation window 21.

The operator blows a gas by blocking the air/liquid supply operation button 26 with a finger.

In the light source 3, the air blowing valve 79 is thereby switched in such a way that the blowing gas from the air blowing pump 78 is supplied to the channel unit portion 43 through the control of the control circuit 80.

The blowing gas from the air blowing valve 79 is transferred to the air blowing input connector 89 via the apparatus-side first air blowing tube 96a, and is supplied from this air blowing input connector 89 to the channel unit 43A.

The blowing gas supplied to the channel unit 43A is supplied to the pressurizing connector receptacle portion 86 via the in-unit air blowing tube 101 and the air-blowing and pressurizing branch portion 102.

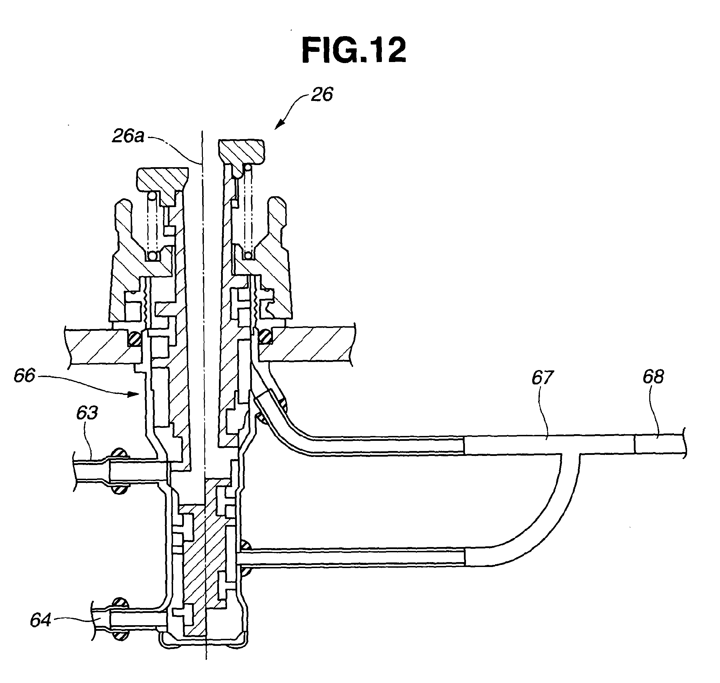

Here, the operator simply blocks the open end of a clearance hole 26a of the air/liquid supply operation button 26 shown in Fig. 12 and does not press down the air/liquid supply operation button 26. Consequently, the air/liquid supply control valve 66 is brought into the condition in which the liquid spraying tube 64 is closed and the air blowing tube 63 is opened. Therefore, the blowing gas supplied to the channel unit 43A is not supplied to the pressurizing connector 47, but is supplied to the pressurizing connector receptacle portion 86.

The blowing gas is supplied from the pressurizing connector receptacle portion 86 of the fluid connector receptacle portion 48 to the pressurizing connector 56 of the fluid connector 53. That is, the blowing gas is supplied from the channel unit portion 43 of the light source 3 to the general connector 29 of the endoscope main body 2.

As described with reference to Fig. 3, the blowing gas supplied to the endoscope main body 2 is passed through the air blowing tube 63, joined with the air/liquid supply tube 68 via the air/liquid supply channel branch portion 67, and is led to the air/liquid supply nozzle 22 of the insertion portion distal end portion 14. The blowing gas is ejected from the opening of the air/liquid supply nozzle 22 toward the surface of the observation window 21 to blow off the adhered materials.

In the case where the soil is not removed, the operator further cleans the surface of the observation window 21 by liquid spraying. The operator presses down the air/liquid supply operation button 26.

The blowing gas supplied to the channel unit 43A is supplied to the pressurizing connector 47 via the in-unit air blowing tube 101 and the air-blowing and pressurizing branch portion 102.

Here, since the air/liquid supply operation button 26 is pressed down, the air/liquid supply control valve 66 is operated in such a way that the air blowing tube 63 in the endoscope main body is closed and the liquid spraying tube 64 is opened, that is, the air blowing channel is closed and the liquid spraying channel is opened. Consequently, the blowing gas supplied to the channel unit 43A is not supplied to the pressurizing connector receptacle portion 86, but is supplied to the pressurizing connector 47.

The blowing gas is supplied from the pressurizing connector 47 into the liquid spraying tank 33 via the pressurizing tube 33aa of the tank tube 33a, pressurizes an air portion in this tank, and pressurizes the liquid surface of the liquid accumulated in the tank. The pressurized liquid is supplied to the channel unit portion 43 of the light source 3 via the liquid guide tube 33ab of the tank tube 33a.

The liquid from the liquid spraying tank 33 is introduced from the liquid spraying connector 46 of the fluid connector receptacle portion 48 into the channel unit 43A, and is led to the liquid spraying connector receptacle portion 87 via the in-unit liquid spraying tube 105.

The liquid is supplied from the liquid spraying connector receptacle portion 87 of the fluid connector receptacle portion 48 to the liquid spraying connector 57 of the fluid connector 53. That is, the sterilized water is supplied from the channel unit portion 43 of the light source 3 to the general connector 29 of the endoscope main body 2.

As described with reference to Fig. 3, the liquid supplied to the endoscope main body 2 is passed through the liquid spraying tube 64, is joined with the air/liquid supply tube 68 via the air/liquid supply channel branch portion 67, and is led to the air/liquid supply nozzle 22 of the insertion portion distal end portion 14. This liquid is ejected from the opening of the air/liquid supply nozzle 22 toward the surface of the observation window 21 to clean the surface of this observation window 21.

In the case where the cleaning of the observation window 21 is judged to be satisfactory, the operator further presses down the air/liquid supply operation button 26 to return the endoscope system 1 to the original state.

In the present embodiment, the air/liquid supply is described as a typical example, whereas explanations of the forward-spraying of liquid and the suction are not provided.

After the endoscopy is completed, the operator removes the endoscope main body 2 to clean and disinfect.

At this time, the operator can remove the endoscope main body 2 simply by removing the general connector 29 of the endoscope main body 2 from the general connector receptacle portion 40 of the light source 3 by one action since the endoscope main body 2 is connected to the light source 3 only.

It is not necessary to remove the tubes and cords 31a to 34a of the suction device 31 and the like, and these may remain connected to the light source 3. The operator cleans and disinfects the endoscope main body 2 in preparation for next endoscopy.

As a result, the endoscope system 1 of the present embodiment has the effect of making it possible to connect the tubes and cords 31a to 34a to the endoscope main body 2 by one action and, thereby, improving the operability.

In the configuration of the present embodiment, the general connector 29 of the endoscope main body 2 is connected to the general connector receptacle portion disposed in the light source 3 serving as an external apparatus.

However, the present invention is not limited to this. A relay unit connecting in between the light source and the endoscope main body 2 may be disposed as an external apparatus, and this relay unit may have a configuration including the general connector receptacle portion.

In the present invention, it is clear that different embodiments in a wide range can be configured based on the present invention without departing from the spirit and scope of the invention. The present invention is defined by the appended claims, and is not limited to specific embodiments thereof.

### Industrial Applicability

As described above, the endoscope system according to the present invention is useful for medical care in applications to intracavital observations, as well as various therapies, treatments, and the like. Furthermore, the endoscope according to the present invention is useful for applications to inspection and the like of flaws, corrosions, and the like in, for example, channels and tanks of various facilities, the insides of bodyworks and wings of aircraft, and the insides of boilers, gas turbine engines, pipelines of chemical plants and the like, and bodies of automobile engines, in addition to the above-described medical care.

### Cross Reference of Related Application

This application claims the benefit of priority based on Japanese Patent Application No. 2003-136392 filed on May 14, 2003, and the contents of which are incorporated in the specification, claims, and drawings of this application.

## Claims

1. An endoscope system **characterized by** comprising:
an endoscope main body having a configuration capable of observing the inside of a subject;
an external apparatus including a light source to emit illumination light for illuminating an inspection area of the subject;
a fluid apparatus to supply a fluid to be ejected from the endoscope main body or to suction a fluid in the endoscope main body, the fluid apparatus disposed separately from the external apparatus;
a connector to connect the endoscope main body to the external apparatus, the connector disposed on one end portion side of the endoscope main body;
a light transmission portion to transmit the light incident on the endoscope main body via the connector to the other end portion side of the endoscope main body, the light transmission portion disposed in the endoscope main body;
a fluid path to allow the fluid to pass through the endoscope main body via the connector, the fluid path disposed in the endoscope main body;
a connector receptacle to connect the endoscope main body to the external apparatus by the connector being inserted into the connector receptacle disposed in the external apparatus;
a light guide portion to guide the illumination light from the light source to the connector receptacle;
a light incident portion which is optically connected to the light guide portion in accordance with an action to insert the connector into the connector receptacle, the light incident portion disposed on one end portion side of the light transmission portion;
a fluid path opening disposed to allow the fluid supplied from the fluid apparatus or the fluid suctioned by the fluid apparatus to flow into the fluid path in accordance with an action to insert the connector into the connector receptacle, the fluid path opening disposed on one end portion side of the fluid path; and
a fluid supply channel which constitutes a flow channel to allow the fluid supplied from the fluid apparatus or the fluid suctioned by the fluid apparatus to flow into the fluid path opening via the external apparatus by being connected to the fluid path opening in accordance with an action to insert the connector into the connector receptacle.

2. The endoscope system according to Claim 1,
**characterized in that**
the fluid supply channel comprises a first connection channel which is connected to the fluid path opening in accordance with an action to insert the connector into the connector receptacle and a second connection channel to connect the first connection channel to the fluid apparatus and to deliver the fluid supplied from the fluid apparatus or the fluid suctioned by the fluid apparatus to the first connection channel.

3. The endoscope system according to Claim 1,
**characterized by** further comprising:
a first electrical connection portion to transmit predetermined electrical signals between the endoscope main body and the external apparatus, the first electrical connection portion disposed on the connecter; and
a second electrical connection portion which is electrically connected to the first electrical connection portion in accordance with an action to insert the connector into the connector receptacle and which allows the electrical signals to be transmitted between the endoscope main body and the external apparatus, the second electrical connection portion disposed on the connector receptacle.

4. The endoscope system according to Claim 2,
**characterized in that**
the first connection channel is disposed in the inside of the external apparatus.

5. The endoscope system according to Claim 2,
**characterized in that**
the first connection channel is disposed in a channel unit having a configuration capable of being removably attached to the external apparatus.

6. The endoscope system according to Claim 4,
**characterized in that**
the first connection channel is disposed in a channel unit having a configuration capable of being removably attached to the external apparatus.

7. The endoscope system according to Claim 2,
**characterized in that**
the first connection channel comprises a first opening which is to be connected to the fluid path opening and which is open in a predetermined direction and a second opening which is to be connected to the second connection channel and which is open in the same direction as that of the first opening.

8. The endoscope system according to Claim 3,
**characterized in that**
the first connection channel comprises a first opening which is to be connected to the fluid path opening and which is open in a predetermined direction and a second opening which is to be connected to the second connection channel and which is open in the same direction as that of the first opening.

9. The endoscope system according to Claim 4,
**characterized in that**
the first connection channel comprises a first opening which is to be connected to the fluid path opening and which is open in a predetermined direction and a second opening which is to be connected to the second connection channel and which is open in the same direction as that of the first opening.

10. The endoscope system according to Claim 5,
**characterized in that**
the first connection channel comprises a first opening which is to be connected to the fluid path opening and which is open in a predetermined direction and a second opening which is to be connected to the second connection channel and which is open in the same direction as that of the first opening.

11. The endoscope system according to Claim 6,
**characterized in that**
the first connection channel comprises a first opening which is to be connected to the fluid path opening and which is open in a predetermined direction and a second opening which is to be connected to the second connection channel and which is open in the same direction as that of the first opening.

12. The endoscope system according to Claim 5,
**characterized in that**
a removable attachment mechanism is disposed to allow the channel unit to be removably mounted to a mounting portion disposed in the external apparatus.

13. The endoscope system according to Claim 6,
**characterized in that**
a removable attachment mechanism is disposed to allow the channel unit to be removably mounted to a mounting portion disposed in the external apparatus.

14. The endoscope system according to Claim 5,
**characterized in that**
the mounting portion disposed in the external apparatus includes an air blowing channel to be supplied with a blowing gas from an air blowing source, the air blowing channel having an air blowing input connector receptacle portion, and
the channel unit has an air blowing input connector to be connected to the air blowing input connector receptacle portion.

15. The endoscope system according to Claim 6,
**characterized in that**
the mounting portion disposed in the external apparatus includes an air blowing channel to be supplied with a blowing gas from an air blowing source, the air blowing channel having an air blowing input connector receptacle portion, and
the channel unit has an air blowing input connector to be connected to the air blowing input connector receptacle portion.

16. The endoscope system according to Claim 2,
**characterized in that**
the first connection channel is a tube channel disposed in the inside of the external apparatus, and
the second connection channel is a tube channel to be connected to the fluid apparatus.

17. The endoscope system according to Claim 7,
**characterized in that**
the first connection channel is a tube channel disposed in the inside of the external apparatus, and
the second connection channel is a tube channel to be connected to the fluid apparatus.

18. The endoscope system according to Claim 16,
**characterized in that**
the first opening is a fluid connector receptacle portion disposed in the external apparatus; and
the second opening is a fluid connector to be connected to the fluid apparatus.

19. The endoscope system according to Claim 18,
**characterized in that**
the fluid connector receptacle portion includes a liquid forward-spraying connector receptacle portion, a suction connector receptacle portion, a pressurizing connector receptacle portion, and a liquid spraying connector receptacle portion, and
the fluid connector includes a liquid forward-spraying connector, a suction connector, a pressurizing connector, and a liquid spraying connector.
